# EUROPEAN PATENT SPECIFICATION

(11) **EP 1 933 773 B1**
(45) Date of publication and mention of the grant of the patent: **08.09.2010**
(21) Application number: 06779888.4
(22) Date of filing: 21.07.2006
(51) Int. Cl.: A61F 2/44, A61B 17/70

(54) **APPARATUS FOR THE NEUROSURGICAL-ORTHOPEDIC TREATMENT OF HUMAN SPINAL COLUMN PATHOLOGIES**
GERÄT ZUR NEUROCHIRURGISCH-ORTHOPÄDISCHEN BEHANDLUNG VON PATHOLOGISCHEN ZUSTÄNDEN DER MENSCHLICHEN WIRBELSÄULE
APPAREIL POUR LE TRAITEMENT ORTHOPÉDIQUE NEUROCHIRURGICAL DES PATHOLOGIES DE LA COLONNE VERTÉBRALE HUMAINE

(30) Priority: 28.07.2005 IT PD20050231
(43) Date of publication of application: 25.06.2008
(73) Proprietor: 2B1 S.r.l., 20124 Milano MI (IT)
(72) Inventor: MIGLIETTA, Carlo, I-20124 Milano MI (IT); TEDESCO, Giuseppe, I-73048 Nardo'Le (IT)
(74) Representative: Iannone, Carlo Luigi
(86) International application number: PCT/IB2006/002004
(87) International publication number: WO 2007/012940

(56) References cited:
- WO-A-00/40179
- FR-A- 2 861 286
- US-A1- 2002 120 335
- US-A1- 2005 131 548
- US-B1- 6 200 347

## Description

The present invention refer to an apparatus for the neurosurgical-orthopedic treatment of human spinal column pathologies, which is particularly, yet not exclusively, useful in treating the lumbar and cervical spinal stenosis.

The lumbar and cervical stenosis is a pathology that affects the human being prevalently with advancing age. This pathology consists in a progressive reduction of the radicular inter-spinal channels existing between adjacent spinal processes of the spinal column in the lumbar, thorax and cervical region. This progressive reduction substantially reduces the space available for the passage of the blood vessels and nervous systems, leading to the following symptoms characteristic of this pathology:
- muted or painful back ache diffusing from the legs;
- numbness and formication of the legs, hind-legs and buttocks;
- weakness, loss of balance and
- falling endurance of physical activities.

These symptoms grow for instance after a walk or after the subject has remained standing for prolonged periods.

In the current medical practice, stenosis is treated in a first phase by applying non-invasive therapies such as medications, gymnastics and appropriate drugs. In a second phase it is necessary to take surgical action by implanting appropriate devices between adjacent spinal processes, which serve the purpose of enlarging and maintaining the enlarged shape of the damaged spinal channels. The subject affected by stenosis thus fully overcomes his pathological condition and recovers the normal functions associated with the spinal column.

A first type of known apparatus is made in a single piece, formed of an elastically yielding material and presents a characteristic, approximately "H" shaped form. The central horizontal element of this apparatus operates as an elastically yielding contrast means to be fitted into an inter-spinal channel between a pair of adjacent spinal processes for the purpose of contrasting the stenosis of the channel. The vertical side elements of this apparatus act as constraining means associated to the contrast means to be bound to the adjacent spinal processes so as to hinder shifting motions of the contrast means outside/inside the inter-spinal channel.

The implanting methodology of this first type of apparatus comprises a first phase of cutting the patient's rear ligament corresponding to the inter-spinal channel affected by the stenosis, and a second phase of emplacing the apparatus.

The main drawback of this apparatus consists in the fact that its implantation is particularly invasive, because in attempting to resolve the patient's pathology it is necessary to take drastic action on a healthy organ of the same. The introduction of the central horizontal element into the inter-spinal channel in fact demands the cutting of the rear ligament before introducing the same.

A second drawback of this device consists in the fact that the material employed for realizing this apparatus, generally a gel, tends to degrade in time and therefore to lose its mechanical features in time. This fact occasions numerous problems associated with the need of replacing the apparatus at regular, approximately pre-established time intervals.

A second known apparatus is essentially formed in two mutually coupled pieces made of a rigid material. In an assembled form, this apparatus defines a rigid contrast means to be fitted into an inter-spinal channel between a pair of adjacent spinal processes, so as to contrast the stenosis of the channel, and constraining means associated with the contrast means to keep the contrast means from shifting outside/inside the inter-spinal channel. In a disassembled form, this apparatus is composed of a first piece formed by the contrast means and first constraining means and by a second means formed in turn by second constraining means.

The implanting methodology of this second type of apparatus comprises a first place of emplacing the first piece of the apparatus on one side of the column, a second phase of emplacing the second piece of the apparatus on the other side of the column corresponding to the first piece, and a third phase of mutually coupling the two pieces.

The feature of this apparatus that looks more advantageous than the above type consists in the fact that its implantation is much less invasive, as no cuts of the rear ligament are needed.

The main drawback of this second type of apparatus consists in the fact that the contrast means provides no form of elastic reaction to the stresses the spinal processes are subjected to. This fact leads to abnormal stresses on the spinal processes.

A further example of apparatus for neurosurgical- orthopedic treatment of human spinal column pathologies is known from patent application US 2002/0120335 A1, which discloses an implant for maintaining a distance between cut spinal bones. The implant has hollow regions for packing osteogenic material. The implant ends have surface projections to reduce slippage. Implants made of bone allograft also have spine contacting ends made of demineralized bone to speed fusion of spine and implant; they may also have bone flaps to fix the implant to the spine.

A disadvantage with the apparatuses known from the state of the art, is that they have not elastic properties and also they provide complex assembling.

The aim of the present invention is to achieve an apparatus for the neurosurgical-orthopedic treatment of human spinal column pathologies capable of resolving the mentioned drawbacks.

Within the abovementioned aim, an object of the invention consists in setting up an apparatus capable of developing a graduated contrast reaction against the stenosis of the channel receiving such apparatus.

A further object of the invention is to set up an apparatus wherein said graduated contrast reaction is mainly induced by the shape of the elastically yielding contrast means.

Another object of the invention is to set up an apparatus whose contrast reaction is enduring.

Not the last object of the invention consists in setting up an apapratus easy to use on the whole and flexible to employ, so as to ease the implaning operations.

This aim, these and other objects that will be better apparent in the following, are achieved by an apparatus for the neurosurgical-orthopedic treatment of human spinal column pathologies according to the enclosed claims.

According to first features of the invention, the elastically yielding contrast means is formed by distancing means which distance a pair of adjacent spinal processes and by elastically yielding connecting means which connect the distancing means, so as to achieve a graduated contrast reaction against the stenosis of the channel the apparatus is applied to.

According to second features of the invention, the distancing means are made of a pair of wings and the elastically yielding connecting means are constituted of an extension common to said distancing means, so that on the whole the wings and the extension form a "C" shaped body that represents a form suitable for performing said graduated elastic reaction.

According to other features of the invention, said "C" shaped body can be made of a metallic or alloy material to guarantee an enduring reaction.

According to yet further features of the invention, the apparatus is in the overall characterized by the fact of being easy to use and flexible to apply, so as to substantially ease the implantation.

Further features and advantages will be better apparent from the description of a preferred embodiment of the apparatus for the neurosurgical-orthopedic treatment of human spinal column pathologies, as shown for indicative yet not limitative purposes in the enclosed drawings, wherein:

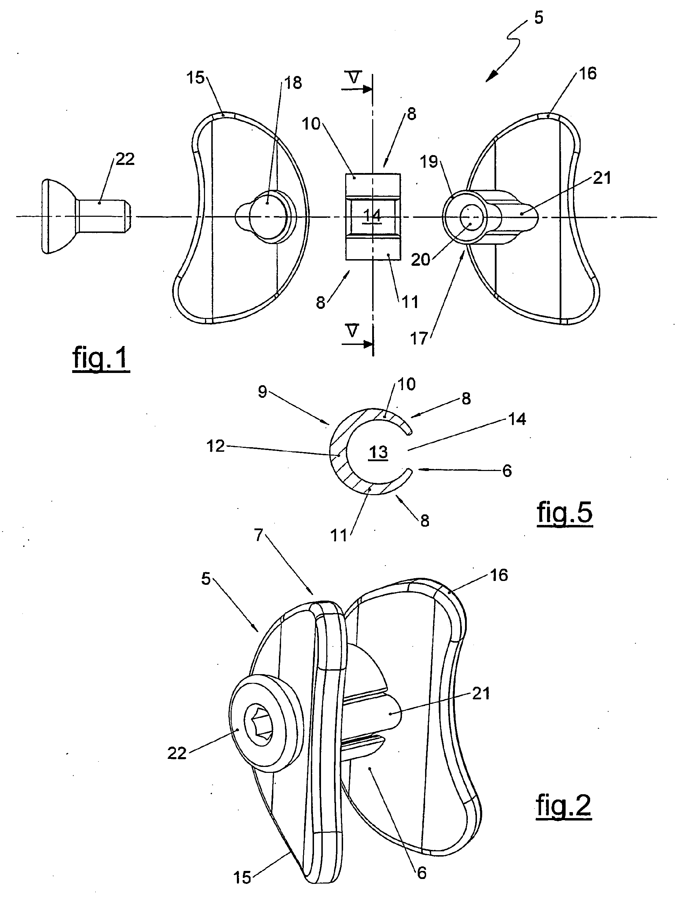

- Figure 1 represents an exploded frontal view of the apparatus according to the invention, in a disassembled condition;
- Figure 2 represents a prospective lateral/frontal view of the apparatus of the previous figure, in an assembled condition;
- Figure 3 represents a detailed view of a first mode of implant of the apparatus of Figure 1 and 2;
- Figure 4 represents a detailed view of a second mode of implant of the apparatus of Figure 1 and 2;
- Figure 5 represents a transversal sectional view, according to the tracing plane V-V of Figure 1, of a part of the apparatus.

With reference to the enclosed drawings, the apparatus for the neurosurgical-orthopedic treatment of human spinal column pathologies, according to the invention, is indicated as a whole by the reference number 5. This apparatus 5 comprises an elastically yielding contrast means indicated by the reference number 6. This contrast means 6 is fitted inside an inter-spinal channel C between a pair of spinal processes P1 and P2 for the purpose of contrasting the stenosis of the channel C. The apparatus further comprises constraining means designated as a whole by the reference number 7, associated with the contrast means 6. These contrast means 7 are constrained to the spinal processes P1 and P2, so as to impede shifting motions of the contrast means 6 outside/inside the inter-spinal channel C. The contrast means is in turn constituted of distancing means, indicated as a whole by the reference number 8, which serve the purpose of distancing the pair of spinal processes P1 and P2 and by elastically yielding connecting means, indicated as a whole by the reference number 9, which have the function of connecting the mentioned distancing means 8. In this manner, the distancing means 8 and the connecting means 9 achieve a contrast reaction graduated to the stenosis of the channel C. These distancing means 8 are appropriately disposed facing each other and placed in contact with the spinal processes P1 and P2. In detail, said distancing means 8 are constituted of a pair of wings designated by the reference numbers 10 and 11. These wings 10 and 11 are advantageously formed in a curved manner and placed in contact with the spinal processes P1 and P2 in the regions of maximum curvature. The connecting means 9 are in turn constituted of an extension 12 common to both distancing means 8. The peculiar characteristic of this extension consists in a thickness growing in a direction toward their central portion. This characteristic determines a variation of the elastic reaction depending on the opposite pressure and traction stresses applied to the distancing means 8. In the overall, therefore, the wings 10 and 11 together with the extension 12 essentially define a "C" type body which, in turn, defines a central cylindrical space 13 and an opening 14 of the space 13 communicating with the exterior. This "C" type body is advantageously realized by employing for instance a metallic alloy belonging to the family of titanium alloys that present fair biocompatibility characteristics, fair elastic properties and high degradation resistance. The constraining means 7 are in turn constituted of a pair of platelets 15 and 16 placed facing each other, and of a connecting element 17 that connects the mentioned platelets 15 and 16. The latter are profiled approximately to the shape of a laid-down "V" and fitted with their opposite extremities bent in a divergent manner. This particular configuration of the platelets 15 and 16 reproduces the lateral profile of the spinal processes P1 and P2 so as to adapt themselves to them once the apparatus 5 has been implanted. Moreover, the platelet designated by the reference number 15 is fitted with a hole 18 provided next to the apex of the same platelet 15. The connecting element 17 is in turn formed by a cylindrical appendix 19 that extends in an orthogonal direction from the platelet 16, devoid of a hole, to the zone next to its apex toward the other platelet, for a length not inferior to that of the contrast means 6. Said connecting means 17 is also fitted with a central hole 20 applied in an axial direction on the appendix 19 and internally threaded, and provided with a bump 21 that extends in a frontal direction from the mantle of the mentioned appendix 19. This appendix 19 is also received inside a central cylindrical space 13 and the bump 21 projects to the outside across the mentioned opening 14. In conclusion, said connecting element 17 comprises a locking key 22 inserted into the hole 18 provided in the platelet 15 and threaded into the central hole 20 provided on the appendix 19.

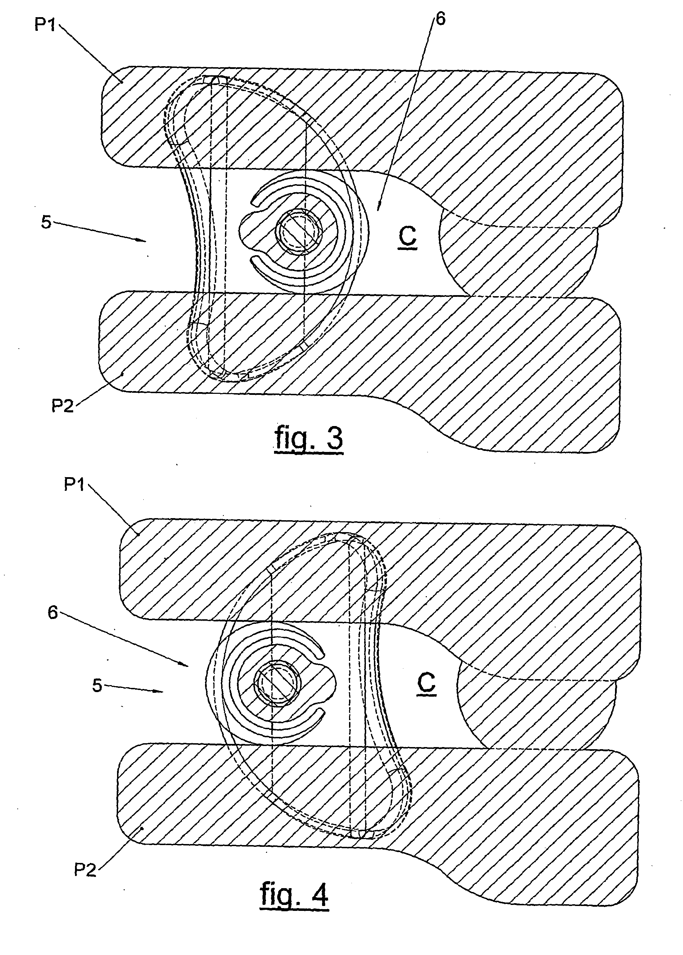

With particular reference to the Figures 3 and 4, the procedure of implanting the apparatus 5 occurs as follows. The first stage consists in preparing the patient for the implantation. The second phase consists in selecting the contrast means 6 among a series of contrast means 9 of different size, depending on the size of the patient's inter-spinal channel C. The third phase consists in introducing the contrast means 6 inside the inter-spinal channel 6 while disposing it transversally to the inter-spinal channel C and according to one of the configuration s shown in the Figures 3 and 4. The fourth phase consists in the complete lateral introduction of the connecting element 17 into the central cylindrical space 13. The fifth phase consists in the final assembly of the apparatus 5, wherein the platelet 15 is coupled to the connecting element 17, the introduction of the locking screw 22 into the central hole 18 of the platelet 15, and the complete tightening down of the screw 22 into the central hole 20.

It has in practice been found that the apparatus for the treatment of neurosurgical-orthopedic pathologies of the human spinal column achieves the pre-established aim and objects. In particular, the apparatus has proved to be capable of performing a graduated contrast reaction against the stenosis of the channel the apparatus is applied to. Moreover, it has been found that graduated contrast reaction performed by the elastically yielding contrast means is above all due to its form. It has further been found that the graduated contrast reaction performed by the elastically yielding contrast means is durable. Finally, the apparatus has on the whole been found easy to use and flexible to employ, so as to ease the implanting operations.

The structure according to the invention is susceptible to numerous modifications and variants, all of which fall within the scope of the same inventive concept.

In a practical embodiment, the materials employed, the forms, dimensions and executive details may differ from those indicated above, but be technically equivalent to them, without thereby abandoning the scope of the invention.

## Claims

1. Apparatus (5) for the neurosurgical-orthopedic treatment of human spinal column pathologies, comprising
an elastically yielding contrast means (6) suitable to fit into an inter-spinal channel (C) between a pair of adjacent spinal processes (P1, P2) for the purpose of contrasting the stenosis of said channel (C), said contrast means (6) comprising in its turn distancing means (8) to distance said pair of spinal processes (P1, P2) and elastically yielding connecting means (9) suitable to connect said distancing means (8), and
constraining means (7) associated with said contrast means (6) to be constrained to said pair of adjacent spinal processes (P1, P2), so as to hinder said contrast means (6) from shifting outside/inside said inter-spinal channel (C),
**characterizing in that**
said distancing means (8) are constituted of a pair of wings (10,11),
said elastically yielding connecting means (9) are constituted of an extension (12) common to both said distancing means (8), which has a growing thickness in the direction toward the central region,
so that said pair of wings (10, 11) and said extension (12) essentially define a "C" shaped cross section body that in turn defines a central cylindrical space (13) and an opening (14) to communicate with the outside,
in order to achieve a graduated contrasting reaction against the stenosis of said channel (C).

2. Apparatus (5), according to claim 1, **characterized in that** said distancing means (8) are arranged to mutually face each other and be placed in contact with said spinal processes (P1, P2).

3. Apparatus (5), according to anyone of the preceding claims, **characterized in that** said wings (10, 11) are shaped in a curved manner.

4. Apparatus (5), according to anyone of the preceding claims, **characterized in that** said "C" shaped body is realized of a material whose physical-chemical characteristics are durable and constant in time.

5. Apparatus (5), according to anyone of the preceding claims, **characterized in that** said constraining means (7) comprise a pair of platelets (15, 16) arranged to face each other and a connecting element (17) to connect said platelets (15,16).

6. Apparatus (5), according to claim 5, **characterized in that** said platelets (15, 16) are shaped approximately like a laid-down "V" and are provided with opposed terminal regions bent in a divergent fashion.

7. Apparatus (5), according to anyone of claims 5 or 6, **characterized in that** one platelet (15) of said pair of platelets (15, 16) comprises a hole (18) provided next to the apex of the same platelet (15).

8. Apparatus (5), according to anyone of claims 5 - 7, **characterized in that** said connecting element (17) comprises a cylindrical appendix (19) extending in an orthogonal direction from the region next to the apex of said platelet (16) devoid of said hole toward the other platelet and has a length not inferior to that of said contrast means (6).

9. Apparatus (5), according to anyone of claims 5 - 8, **characterized in that** said connecting element (17) is fitted with a central hole (20) provided in an axial direction on said appendix (19) and internally threaded, and provided with a bump (21) that extends frontally from the mantle of said appendix (19).

10. Apparatus (5), according to anyone of claims 8 or 9, **characterized in that** said appendix (19) is received inside said space obtained in said contrast means (6) and said bump (21) projects toward the outside across said opening (14).

11. Apparatus (5), according to anyone of claims 5 - 10, **characterized in that** said connecting element (17) comprises a locking screw (22) inserted in said hole (18) on said platelet (15) and is 10 engaged in said central hole (20) by threading ft into said central hole (20) in said appendix (19).

12. Apparatus (5), according to one anyone of the preceding claims, **characterized in that** said wings (10, 11) are suitable to be placed in contact with said spinal processes (P1, P2) in the regions of maximum curvature.

13. Apparatus (5), according to anyone of the preceding claims, **characterized in that** said "C" shaped body is suitable to be arranged with said communication opening (14) turned to the opposite side of said spinal column and that or with said communicating opening (14) turned toward said spinal column.

## Patentansprüche

1. Vorrichtung (5) für die neurochirurgisch-orthopädische Behandlung von Pathologien der menschlichen Wirbelsäule, umfassend:
ein elastisch nachgebendes Kontrastmittel (6), das geeignet ist, um zwischen einem Paar von benachbarten Dornfortsätzen (P1, P2) in einen Zwischenwirbelkanal (C) zu dem Zweck der Kontrastbildung der Stenose des Kanals (C) zu passen, wobei das Kontrastmittel (6) zur Beabstandung des Paars der Dornfortsätze (P1, P2) Abstandsmittel (8) und ein zur Verbindung der Abstandsmittel (8) geeignetes, elastisch nachgebendes Verbindungsmittel (9) aufweist, und
ein Beschränkungsmittel (7), das dem Kontrastmittel (6) zugeordnet ist, das auf das Paar der benachbarten Dornfortsätze (P1, P2) beschränkt ist, um das Kontrastmittel an einer Verschiebung aus dem/in den Zwischenwirbelkanal (C) zu hindern,
**dadurch gekennzeichnet, dass**
die Abstandsmittel (8) aus einem Paar Flügel (10, 11) bestehen,
das elastisch nachgebende Verbindungsmittel (9) aus einem Ansatzstück (12) besteht, das den beiden Abstandsmitteln (8) gemeinsam ist, welches in der Richtung auf den Zentralbereich eine ansteigende Dicke aufweist,
so dass das Paar der Flügel (10, 11) und das Ansatzstück (12) einen im Querschnitt im Wesentlichen "C"-förmigen Körper definieren, der wiederum einen mittigen zylindrischen Raum (13) und eine Öffnung (14) definiert, um mit der Außenseite in Verbindung zu stehen,
um eine abgestufte Kontrastierungsreaktion gegen die Stenose des Kanals (C) zu erzielen.

2. Vorrichtung (5) gemäß Anspruch 1, **dadurch gekennzeichnet, dass** die Abstandsmittel (8) angeordnet sind, um sich wechselseitig gegenüber zu liegen und in Kontakt mit den Dornfortsätzen (P1, P2) gebracht zu werden.

3. Vorrichtung (5) gemäß einem der vorangehenden Ansprüche, **dadurch gekennzeichnet, dass** die Flügel (10, 11) in gekrümmter Weise geformt sind.

4. Vorrichtung (5) gemäß einem der vorangehenden Ansprüche, **dadurch gekennzeichnet, dass** der "C"-förmige Körper aus einem Material realisiert ist, dessen physikalisch-chemische Eigenschaften dauerhaft und zeitlich konstant sind.

5. Vorrichtung (5) gemäß einem der vorangehenden Ansprüche, **dadurch gekennzeichnet, dass** das Beschränkungsmittel (7) ein Paar Plättchen (15, 16) aufweist, die angeordnet sind, um einander gegenüberzuliegen sowie ein Verbindungselement (17), um die Plättchen (15, 16) zu verbinden.

6. Vorrichtung (5) gemäß Anspruch 5, **dadurch gekennzeichnet, dass** die Plättchen (15, 16) annähernd wie ein liegendes "V" geformt sind und mit gegenüberliegenden Anschlussbereichen versehen sind, die in divergierender Weise gebogen sind.

7. Vorrichtung (5) gemäß einem der Ansprüche 5 oder 6, **dadurch gekennzeichnet, dass** das Plättchen (15) des Paars der Plättchen (15, 16) eine Öffnung (18) aufweist, die neben dem Scheitel des Plättchens (15) vorgesehen ist.

8. Vorrichtung (5) gemäß einem der Ansprüche 5 - 7, **dadurch gekennzeichnet, dass** das Verbindungselement (17) einen zylindrischen Fortsatz (19) aufweist, der sich in rechtwinkliger Richtung von dem Bereich neben dem Scheitel des Plättchens (16) ohne das Loch in Richtung auf das andere Plättchen erstreckt und eine Länge aufweist, die nicht geringer ist als diejenige des Kontrastmittels (6).

9. Vorrichtung (5) gemäß einem der Ansprüche 5 - 8, **dadurch gekennzeichnet, dass** das Verbindungselement (17) mit einem Mittelloch (20) versehen ist, das in einer Axialrichtung auf dem Fortsatz (19) versehen und innen mit einem Gewinde versehen ist und mit einer Erhebung (21) versehen ist, die sich an der Frontseite von dem Mantel des Fortsatzes (19) erstreckt.

10. Vorrichtung (5) gemäß einem der Ansprüche 8 oder 9, **dadurch gekennzeichnet, dass** der Fortsatz (19) in dem in dem Kontrastmittel (6) enthaltenen Raum aufgenommen wird und die Erhebung (21) quer durch die Öffnung (14) in Richtung nach außen hervorsteht.

11. Vorrichtung (5) gemäß einem der Ansprüche 5 - 10, **dadurch gekennzeichnet, dass** das Verbindungselement (17) eine Arretierschraube (22) aufweist, die in das Loch (18) auf dem Plättchen (15) eingesetzt ist und die 10 eingreift in die Zentralbohrung (20) durch eine Schraubpassung in der Zentralbohrung (20) in dem Fortsatz (19).

12. Vorrichtung (5) gemäß einem der vorangehenden Ansprüche, **dadurch gekennzeichnet, dass** die Flügel (10, 11) geeignet sind, um in Kontakt mit den Dornfortsätzen (P1, P2) in den Bereichen maximaler Krümmung gebracht zu werden.

13. Vorrichtung (5) gemäß einem der vorangehenden Ansprüche, **dadurch gekennzeichnet,**
**dass** der "C"-förmige Körper geeignet ist, um angeordnet zu werden mit seiner Verbindungsöffnung (14) zugewandt zu der gegenüberliegenden Seite der Wirbelsäule und
**dass** die oder mit der Verbindungsöffnung (14) gerichtet ist in Richtung auf die Wirbelsäule.

## Revendications

1. Appareil (5) pour le traitement neurochirurgical-orthopédique des pathologies de la colonne vertébrale humaine, comprenant
un moyen d'opposition élastiquement déformable (6) pouvant être ajusté dans un canal interépineux (C) entre une paire d'apophyses épineuses adjacentes (P1, P2) dans le but de s'opposer à la sténose dudit canal (C), ledit moyen d'opposition (6) comprenant quant à lui des moyens d'espacement (8) pour espacer ladite paire d'apophyses épineuses (P1, P2) et des moyens de raccordement élastiquement déformables (9) pouvant relier lesdits moyens d'espacement (8), et
des moyens de contrainte (7) associés audit moyen d'opposition (6) devant être contraints sur ladite paire d'apophyses épineuses (P1, P2), de façon à empêcher ledit moyen d'opposition (6) de se déplacer à l'extérieur/intérieur dudit canal interépineux (C).
**caractérisé en ce que**
lesdits moyens d'espacement (8) sont constitués d'une paire d'ailes (10, 11).
lesdits moyens de raccordement élastiquement déformables (9) sont constitués d'une extension (12) commune aux deux moyens d'espacement (8), qui a une épaisseur croissante en direction de la région centrale,
ainsi, ladite paire d'ailes (10, 11) et ladite extension (12) définissent essentiellement un corps à section transversale en forme de "C", qui définit quant à lui un espace cylindrique central (13) et une ouverture (14) pour communiquer avec l'extérieur,
afin de parvenir à une réaction progressive d'opposition à la sténose dudit canal (C).

2. Appareil (5), selon la revendication 1, **caractérisé en ce que** lesdits moyens d'espacement (8) sont disposés de façon à se faire face et à être placés en contact avec lesdites apophyses épineuses (P1, P2).

3. Appareil (5), selon l'une quelconque des revendications précédentes, **caractérisé en ce que** lesdites ailes (10, 11) ont une forme courbée.

4. Appareil (5), selon l'une quelconque des revendications précédentes, **caractérisé en ce que** ledit corps en forme de "C" est réalisé en un matériau dont les caractéristiques physico-chimiques sont durables et constantes dans le temps.

5. Appareil (5), selon l'une quelconque des revendications précédentes, **caractérisé en ce que** lesdits moyens de contrainte (7) comprennent une paire de plaquettes (15, 16) disposées de façon à se faire face et un élément de raccordement (17) pour relier lesdites plaquettes (15, 16).

6. Appareil (5), selon la revendication 5, **caractérisé en ce que** lesdites plaquettes (15, 16) sont façonnées approximativement à la manière d'un "V" allongé et sont munies de régions terminales courbées de façon divergente.

7. Appareil (5), selon l'une quelconque des revendications 5 ou 6, **caractérisé en ce qu'**une plaquette (15) de ladite paire de plaquettes (15, 16) comprend un trou (18) situé près du sommet de la même plaquette (15).

8. Appareil (5), selon l'une quelconque des revendications 5 à 7, **caractérisé en ce que** ledit élément de raccordement (17) comprend un appendice cylindrique (19) s'étendant dans une direction orthogonale à partir de la région proche du sommet de ladite plaquette (16) dépourvue dudit trou, vers l'autre plaquette, et a une longueur non inférieure à celle dudit moyen d'opposition (6).

9. Appareil (5), selon l'une quelconque des revendications 5 à 8, **caractérisé en ce que** ledit élément de raccordement (17) est pourvu d'un trou central (20) formé dans une direction axiale dans ledit appendice (19) et présentant un filetage interne, et pourvu d'une protubérance (21) qui s'étend vers l'avant à partir du manteau dudit appendice (19).

10. Appareil (5), selon l'une quelconque des revendications 8 ou 9, **caractérisé en ce que** ledit appendice (19) est reçu à l'intérieur dudit espace obtenu dans ledit moyen d'opposition (6) et ladite protubérance (21) fait saillie vers l'extérieur à travers ladite ouverture (14).

11. Appareil (5), selon l'une quelconque des revendications 5 à 10, **caractérisé en ce que** ledit élément de raccordement (17) comprend une vis de blocage (22) insérée dans ledit trou (18) formé dans ladite plaquette (15) et est engagé dans ledit trou central (20) en étant vissé dans ledit trou central (20) dudit appendice (19).

12. Appareil (5), selon l'une quelconque des revendications précédentes, **caractérisé en ce que** lesdites ailes (10, 11) sont adaptées pour être mises en contact avec les apophyses épineuses (P1, P2) dans les régions de courbure maximale.

13. Appareil (5), selon l'une quelconque des revendications précédentes, **caractérisé en ce que** ledit corps en forme de "C" est adapté pour être disposé avec ladite ouverture de communication (14) tournée du côté opposé à ladite colonne vertébrale et/ou avec ladite ouverture de communication (14) tournée vers ladite colonne vertébrale.
